# EUROPÄISCHE PATENTSCHRIFT

(11) **EP 1 891 993 B1**
(45) Veröffentlichungstag und Bekanntmachung des Hinweises auf die Patenterteilung: **05.10.2011**
(21) Anmeldenummer: 07015669.0
(22) Anmeldetag: 09.08.2007
(51) Int. Cl.: A61L 31/10, A61L 31/02, A61L 31/14

(54) **Biokorrodierbares metallisches Implantat mit einer Beschichtung oder Kavitätenfüllung aus einem Peg/Plga-Copolymer**
Bio-corrodible metal implant with a coating or cavity filling made of a Peg/Plga-copolymer
Implant métallique biocorrodable doté d'un revêtement ou d'un remplissage de cavité en copolymère Peg/Plga

(30) Priorität: 22.08.2006 DE 102006039346
(43) Veröffentlichungstag der Anmeldung: 27.02.2008
(73) Patentinhaber: Biotronik VI Patent AG, 6341 Baar (CH)
(72) Erfinder: Borck, Alexander, Dr., 91086 Aurachtal (DE)
(74) Vertreter: Lindner-Vogt, Karin L.

(56) Entgegenhaltungen:
- EP-A- 1 600 182
- US-A1- 2002 004 060
- US-A1- 2004 034 409

## Beschreibung

Die Erfindung betrifft (i) ein Implantat aus einem biokorrodierbaren metallischen Werkstoff, das eine Beschichtung oder Kavitätenfüllung aufweist, die aus einem Polyethylenglycol/Poly(D,L-lactid-co-glycolid)-Copolymer (PEG/PLGA-Copolymer) besteht oder dieses enthält, sowie (ii) eine neue Verwendung des PEG/PLGA-Copolymers.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Um unnötige Gefäßbeschädigungen zu vermeiden, sollte der Stent nach dem Aufweiten und nach Entfernen des Ballons nicht oder nur in geringem Umfang elastisch zurückfedern, so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt. In der Praxis wird zur Realisation der genannten mechanischen Eigenschaften der Stent in der Regel aus einem metallischen Werkstoff geformt.

Neben den mechanischen Eigenschaften eines Stents sollte dieser aus einem biokompatiblen Material bestehen, um Abstoßungsreaktionen vorzubeugen. Derzeit werden bei etwa 70% aller perkutanen Interventionen Stents eingesetzt, in 25% aller Fälle kommt es jedoch zu einer In-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatz zur Minderung der Restenoserate sieht vor, auf dem Stent eine pharmazeutisch aktive Substanz (Wirkstoff) bereitzustellen, die den Mechanismen der Restenose entgegenwirkt und den Heilungsverlauf unterstützt. Der Wirkstoff wird in Reinform oder eingebettet in eine Trägermatrix als Beschichtung aufgetragen oder in Kavitäten des Implantats gefüllt. Beispiele umfassen die Wirkstoffe Sirolimus und Paclitaxel.

Ein weiterer aussichtsreicher Ansatz zur Lösung des Problems liegt in der Verwendung biokorrodierbarer Metalle und deren Legierungen, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium > 90%, Yttrium 3,7 - 5,5%, Seltenerdmetallen 1,5 - 4,4% und Rest < 1 % bekannt, die sich insbesondere zur Herstellung einer Endoprothese, z. B. in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen.

Die Kombination aus Wirkstofffreisetzung und biokorrodierbarem metallischen Wirkstoff erscheint besonders aussichtsreich. Der Wirkstoff wird als Beschichtung aufgebracht oder in eine Kavität im Implantat eingebracht - zumeist eingebettet in eine Trägermatrix. Bekannt sind beispielsweise Stents aus einer biokorrodierbaren Magnesiumlegierung mit einer Beschichtung aus einem Poly(L-lactid). Ungeachtet der erreichten Fortschritte sind jedoch noch folgende Probleme zu lösen:
Die Abbauprodukte der Trägermatrix sollen keinen merklichen Einfluss auf den lokalen pH-Wert besitzen, um einerseits ungewünschte Gewebsreaktionen zu vermeiden und andererseits den Einfluss auf den Korrosionsprozess des metallischen Implantatswerkstoffs zu mindern.

Der Erfindung liegt die Aufgabe zugrunde, die geschilderten Nachteile des Standes der Technik zu überwinden.

Ein erster Aspekt der Erfindung liegt in der Bereitstellung eines Implantats aus einem biokorrodierbaren metallischen Werkstoff und mit einer Beschichtung oder Kavitätenfüllung bestehend aus oder enthaltend ein Diblock- oder Triblock-Copolymer aus
(i) einem Poly(D,L-lactid-co-glycolid)-Block und
(ii) einem Polyethylenglycol-Block.

Das erfindungsgemäße PEG/PLGA-Copolymer zeigt einen initalen Abbau im Polyethylenglycol-Block. Der Poly(D,L-lactid-co-glycolid)-Block ist deutlich abbaustabiler. Beim Abbau entstehen Hydroxyl-Gruppen, die ihrer chemischen Natur wegen allenfalls einen geringfügigen Effekt auf den lokalen pH-Wert haben. Eine Trägermatrix aus Polylactid hydrolisiert dagegen unter Ausbildung von Säurefunktionen, die für Gewebsreaktionen, wie Entzündungen, verantwortlich gemacht werden. Neben dem positiven Einfluss auf das Gewebe sind Hydroxyl-Gruppen für den Grundkörper geeigneter, insbesondere, wenn dieser aus Magnesium und seinen Legierungen besteht, da sie den Abbau nicht zusätzlich beschleunigen.

Der schnelle Abbau des Polyethylenglycol-Blocks führt ferner zu einer deutlichen Steigerung der Porosität der Trägermatrix, so dass der Abbau des biokorrodierbaren metallischen Implantatswerkstoffs weniger durch die Gegenwart der Trägermatrix beeinflusst wird.

Eine Beschichtung im Sinne der Erfindung ist eine zumindest abschnittsweise Auftragung der Komponenten auf den Grundkörper des Implantats, insbesondere Stents. Vorzugsweise wird die gesamte Oberfläche des Grundkörpers des Implantats/Stents von der Beschichtung bedeckt. Alternativ kann das PEG/PLGA-Copolymer in einer Kavität des Implantats/Stents bereitgestellt werden.

Das erfindungsgemäß eingesetzte PEG/PLGA-Copolymer ist hochgradig biokompatibel und biodegradierbar. Die Verarbeitung kann nach Standverfahren erfolgen. Das Blockpolymer besitzt eine hydrophobe und hydrophile Domäne und eignet sich für die Aufnahme hydrophober als auch hydrophiler Wirkstoffe. Ebenso sind Wirkstoffe mit amphiphilen Charakter in dieser Matrix solubilisierbar. Vorzugsweise eignet sich die Trägermatrix daher zur Inkorporation von Wirkstoffen, die bei Änderung des pH-Werts ihre Lösungseigenschaften ändern (z. B. Wirkstoffe mit Aminfunktionen); einem Problem, dass besonders beim Abbau von Magnesiumlegierungen auftritt. Das Copolymer ist ferner pH-Wert-neutral, so dass sich das Material für die Einbettung von pH-sensitiven Wirkstoffen besonders eignet. Das PEG/PLGA-Copolymer dient somit in der Regel als Trägermatrix für einen pharmazeutischen Wirkstoff, kann ggf. jedoch auch für Fluoreszenz- oder Röntgenmarker oder andere Zusätze enthalten. Diblock- und Triblockcopolymere von PEG/PLGA sind kommerziell unter dem Handelsnamen Resomer bei der Firma Boehringer Ingelheim, Deutschland, erhältlich.

Vorzugsweise hat der Polyethylenglycol-Block ein mittleres Molekulargewicht im Bereich von 4.000 bis 8.000 Dalton.

Ferner ist bevorzugt, wenn der Poly(D,L-lactid-co-glycolid)-Block ein mittleres Molekulargewicht im Bereich von 20.000 bis 120.000 Dalton aufweist.

Als biokorrodierbar im Sinne der Erfindung werden metallische Werkstoffe bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Biokorrodierbare metallische Werkstoffe im Sinne der Erfindung umfassen insbesondere Metalle und Legierungen ausgewählt aus der Gruppe der Elemente Eisen, Wolfram und Magnesium. Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Vorzugsweise ist der biokorrodierbare Werkstoff eine Magnesiumlegierung. Insbesondere enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die metallischen Werkstoffe bzw. Magnesiumlegierungen sind so in ihrer Zusammensetzung zu wählen, dass sie biokorrodierbar sind. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl2 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird.

Ein zweiter Aspekt der Erfindung bezieht sich auf die Verwendung von PEG/PLGA-Coplymeren der zuvor beschriebenen Zusammensetzung als Beschichtungsmaterial für einen Stent aus einem biokorrodierbaren metallischen Werkstoff oder als Füllung für eine Kavität in einem Stent aus einem biokorrodierbaren metallischen Werkstoff.

Stents aus der biokorrodierbaren Magnesiumlegierung WE43 (97 Gew.% Magnesium, 4 Gew.% Yttrium, 3 Gew.% Seltenerdmetalle außer Yttrium) wurden wie folgt beschichtet:
Die Magnesiumoberflächen der Stents wurden durch Behandlung mit einem Argonplasma aufgeraut, um eine höhere Haftigkeit des Wirkstoffs auf der Stentoberfläche zu erzielen. Alternativ oder ergänzend kann eine Oberflächenmodifizierung, z. B. durch Silanisierungen mit Methoxy- oder Ethoxysilanen oder mit Hilfe von Phosphonsäurederivaten das Haftvermögen zum metallischen Grundkörper erhöhen.

Es wurde eine 0,1%ige Lösung des Blockpolymers (Diblock-Copolymer aus Poly(D,L-lactid-co-glycolid)-Block und 15%igen Polyethylenglycol-Block (5.000 Dalton); käuflich erwerbbar unter dem Handelsnamen RESOMER, Type RGP d 50155 bei der Firma Boehringer, Ingelheim) in Chlorform angesetzt Mit einem Airbrushsystem wurde die Lösung auf den Stent gesprüht und dieser dann für 24h bei Raumtemperatur getrocknet.

## Patentansprüche

1. Implantat aus einem biokorrodierbaren metallischen Werkstoff und mit einer Beschichtung oder Kavitätenfüllung bestehend aus oder enthaltend ein Diblock- oder Triblock-Copolymer aus
(i) einem Poly(D,L-lactid-co-glycolid)-Block und
(ii) einem Polyethylenglycol-Block.

2. Implantat nach Anspruch 1, bei dem der biokorrodierbare metallische Werkstoff eine Magnesiumlegierung ist.

3. Implantat nach Anspruch 1, bei der Polyethylenglycol-Block ein mittleres Molekulargewicht im Bereich von 4.000 bis 8.000 Dalton hat.

4. Implantat nach Anspruch 1, bei der Poly(D,L-lactid-co-glycolid)-Block ein mittleres Molekulargewicht im Bereich von 20.000 bis 120.000 Dalton hat.

5. Implantat nach einem der vorhergehenden Ansprüche, bei dem das Implantat ein Stent ist.

6. Verwendung eines Diblock- oder Triblock-Copolymers aus
(i) einem Poly(D,L-lactid-co-glycolid)-Block und
(ii) einem Polyethylenglycol-Block
als Beschichtungsmaterial für einen Stent aus einem biokorrodierbaren metallischen Werkstoff oder als Füllung für eine Kavität in einem Stent aus einem biokorrodierbaren metallischen Werkstoff.

## Claims

1. An implant made of a biocorrodible metallic material and comprising a coating or cavity filling made of or containing a diblock or triblock copolymer comprising
(i) a poly(D,L-lactide-co-glycolide) block and
(ii) a polyethylene glycol block.

2. The implant according to claim 1, wherein the biocorrodible metallic material is a magnesium alloy.

3. The implant according to claim 1, wherein the polyethylene glycol block has a mean molecular weight in the range of 4000 to 8000 daltons.

4. The implant according to claim 1, wherein the poly(D,L-lactide-co-glycolide) block has a mean molecular weight in the range of 20000 to 120000 daltons.

5. The implant according to one of the preceding claims, wherein the implant is a stent.

6. The use of a diblock or triblock copolymer comprising
(i) a poly(D,L-lactide-co-glycolide) block and
(ii) a polyethylene glycol block
as the coating material for a stent made of a biocorrodible metallic material or as a filling for a cavity in a stent made of a biocorrodible metallic material.

## Revendications

1. Implant constitué d'un matériau métallique bio-corrodable, avec un revêtement ou un remplissage de cavité constitué d'un ou contenant un copolymère dibloc ou tribloc à partir de
(i) un bloc poly(D,L-lactide-co-glycolide) et
(ii) un bloc polyéthylène glycol.

2. Implant selon la revendication 1, dans lequel le matériau métallique bio-corrodable est un alliage de magnésium.

3. Implant selon la revendication 1, dans lequel le bloc polyéthylène glycol a un poids moléculaire moyen compris entre 4000 et 8000 Dalton.

4. Implant selon la revendication 1, dans lequel le bloc poly(D,L-lactide-coglycolide) a un poids moléculaire moyen compris entre 20.000 et 120.000 Dalton.

5. Implant selon l'une des revendications précédentes, dans lequel l'implant est un stent.

6. Utilisation d'un copolymère dibloc ou tribloc, constitué de
(i) un bloc poly(D,L-lactide-co-glycolide) et
(ii) un bloc polyéthylène glycol
comme matériau de revêtement pour un stent constitué d'un matériau métallique bio-corrodable, ou comme remplissage pour une cavité dans un stent constitué d'un matériau métallique bio-corrodable.
